# EUROPEAN PATENT APPLICATION

(11) **EP 4 328 243 A1**
(43) Date of publication of application: **28.02.2024**
(21) Application number: 22792028.7
(22) Date of filing: 20.04.2022
(51) Int. Cl.: C07K 16/28, A61P 35/00, A61K 39/00

(54) **ASYMMETRIC ANTIBODY WITH IMPROVED CYTOTOXICITY AGAINST CANCER CELL**

(30) Priority: 20.04.2021 KR 20210050921; 19.04.2022 KR 20220048257
(71) Applicant: Korea University Research and Business Foundation, Seoul 02841 (KR); SG Medical Inc., Songpa-gu Seoul 05548 (KR)
(72) Inventor: JUNG, Sang Taek, Seoul 06217 (KR); LEE, Ji Chul, Gwangmyeong-si Gyeonggi-do 14244 (KR); MIN, Sung-Won, Seoul 04701 (KR); BAE, Gong Deuk, Seoul 05381 (KR); KWON, Hyeong Sun, Seoul 02253 (KR); LEE, Sang Min, Gapyeong-gun Gyeonggi-do 12452 (KR)
(74) Representative: V.O.
(86) International application number: PCT/KR2022/005661
(87) International publication number: WO 2022/225329

(57) **Abstract**

The present disclosure relates to an anti-CD20 asymmetric antibody including a single-chain variable fragment (scFv) on one side and an antigen-binding fragment (Fab) on the other side. Compared to an antibody having a symmetric structure, the asymmetric antibody of the present disclosure effectively activates complements and exhibits significantly improved complement-dependent cytotoxicity (CDC), and thus can be advantageously used as an effective therapeutic agent or therapeutic adjuvant for treating cancer.

## Description

### [Technical Field]

The present disclosure relates to a novel anti-CD20 asymmetric antibody with improved complement-dependent cytotoxicity (CDC) or an antigen-binding fragment thereof and/or a composition for preventing, treating and/or diagnosing cancer using the same.

### [Background Art]

The global anticancer drug market has shown an average annual growth rate of 10-13% over the past decade, and is expected to reach a total of 200 billion dollars by 2022. As of 2020, one out of five men and one out of six women are experiencing caner at least once in their lifetime globally, and one out of eight men and one out of eleven women die from cancer. The morbidity and mortality of cancer are increasing continuously.

The global anticancer drug market is undergoing a paradigm shift from the first-generation chemotherapy to the second-generation targeted anticancer drugs and the third-generation immune anticancer drugs. Departing from the treatment methods aimed simply at reducing or suppressing cancer in the early days, the targeted anticancer treatment technologies that selectively attack cancer cells only without causing damage to the cells that divide normally and rapidly are being researched actively. The development of the targeted anticancer treatment technologies consists of two steps: selection of receptor expressed specifically in cancer cells, which enable selective targeting of cancer cells, and development of targeting compound that bind to the receptors.

Rituximab is a chimeric anti-CD20 monoclonal antibody commercialized for tumor treatment and is the first monoclonal antibody therapeutic agent approved by the US FDA. Monoclonal antibodies against the CD20 antigen were produced from mouse in the early 1980s. While they have superior reactivity with the antigen, they have disadvantages that they have short half-life in vivo and induce antigenicity because they are heterologous antigens (HAMAs, human anti-mouse antibodies). In order to compensate for the shortcomings of the mouse-derived antibodies and increase their biological effects, rituximab, which is a chimeric antibody wherein its variable region responsible for antigen recognition is of mouse origin and the constant region is of human origin, was synthesized through genetic recombination technology.

The CD20 antigen is known to be involved in the cell cycle entry of B cells or differentiation into B cells. Because the CD20 antigen does not shed easily from the cell surface and is not easily modified or incorporated, it is suitable for utilization as a target molecule. The distribution of the CD20 antigen is wide in normal cells, from pre-B cells to activated B cells. But, it is not distributed in other cell lineages such as hematopoietic stem cells and plasma cells. In the case of lymphoma, the CD20 antigen is expressed in most of B-cell lymphoma and chronic lymphocytic leukemia and in 50% of pre-B cell acute lymphocytic leukemia. Therefore, it can be an appropriate target for inducing tumor-specific immune responses using monoclonal antibodies (Maloney DG et al., IDEC-C2B8 (Rituximab) anti-CD20 monoclonal antibody therapy in patients with relapsed low-grade non-Hodgkin's lymphoma. Blood, 90: 2188-2195, 1997; McLaughlin P et al., Rituximab chimeric anti-CD20 monoclonal antibody therapy for relapsed indolent lymphoma: half of patients respond to a four-dose treatment program. J Clin Oncol, 16: 2825-2833, 1998).

However, about half of patients with low-grade lymphoma and about 60-70% of patients with high-grade lymphoma show resistance for rituximab, and there are some reports that the complement-mediated cytotoxicity is reduced when the expression of CD55, CD59, etc. is high. But, it is unclear why a significant number of patients do not respond.

A number of papers and patent documents are referenced throughout the present specification and their citations are indicated. The contents of the cited papers and patent documents are incorporated by reference into the present specification in their entirety to more clearly describe the level of the technical field to which the present disclosure belongs and the contents of the present disclosure.

### [Disclosure]

### [Technical Problem]

The inventors of the present disclosure have made intensive research efforts to develop a therapeutic agent capable of effectively killing cancer cells that do not respond to or show resistance to rituximab. As a result, they have found out that an anti-CD20 asymmetric antibody including scFv on one side and Fab on the other side has significantly improved complement-dependent cytotoxicity (CDC) and have completed the present disclosure.

The present disclosure is directed to providing an anti-CD20 asymmetric antibody with improved complement-dependent cytotoxicity (CDC), which includes a single-chain variable fragment (scFv) and an antigen-binding fragment (Fab), or an antigen-binding fragment thereof.

The present disclosure is also directed to providing a nucleic acid molecule encoding the anti-CD20 asymmetric antibody or an antigen-binding fragment thereof.

The present disclosure is also directed to providing a vector including the nucleic acid molecule.

The present disclosure is also directed to providing a host cell transformed with the vector.

The present disclosure is also directed to providing a pharmaceutical composition for preventing or treating cancer, which contains the anti-CD20 asymmetric antibody or an antigen-binding fragment thereof, a nucleic acid molecule encoding the antibody or an antigen-binding fragment thereof or a vector including the nucleic acid molecule as an active ingredient.

The present disclosure is also directed to providing a method for preventing or treating cancer, which includes a step of administering a pharmaceutically effective amount of the anti-CD20 asymmetric antibody or an antigen-binding fragment thereof, a nucleic acid molecule encoding the antibody or an antigen-binding fragment thereof or a vector including the nucleic acid molecule to a subject.

The present disclosure is also directed to providing a therapeutic use of the anti-CD20 asymmetric antibody or an antigen-binding fragment thereof, a nucleic acid molecule encoding the antibody or an antigen-binding fragment thereof or a vector including the nucleic acid molecule.

The present disclosure is also directed to providing a composition for diagnosing cancer, which contains the anti-CD20 asymmetric antibody or an antigen-binding fragment thereof, a nucleic acid molecule encoding the antibody or an antigen-binding fragment thereof or a vector including the nucleic acid molecule as an active ingredient.

The present disclosure is also directed to providing a method for preparing an anti-CD20 asymmetric antibody with improved complement-dependent cytotoxicity or an antigen-binding fragment thereof, which includes a step of preparing scFv and a step of preparing Fab.

The present disclosure is also directed to providing a method for preparing a nucleic acid molecule encoding an anti-CD20 asymmetric antibody with improved complement-dependent cytotoxicity or an antigen-binding fragment thereof, which includes a step of preparing a nucleic acid molecule encoding scFv and a nucleic acid molecule encoding Fab.

Other purposes and advantages of the present disclosure will become more apparent by the following detailed description, claims and drawings.

### [Technical Solution]

In an aspect, the present disclosure provides a method for preparing a nucleic acid molecule encoding anti-CD20 asymmetric antibody with improved complement-dependent cytotoxicity (CDC) or an antigen-binding fragment thereof, which includes:
(a) a step of preparing a nucleic acid molecule encoding a single-chain variable fragment (scFv), which includes LCDR1 of SEQ ID NO 16, LCDR2 of SEQ ID NO 18, LCDR3 of SEQ ID NO 20, HCDR1 of SEQ ID NO 24, HCDR2 of SEQ ID NO 26 and HCDR3 of SEQ ID NO 28; and
(b) a step of preparing a nucleic acid molecule encoding an antigen-binding fragment (Fab), which includes LCDR1 of SEQ ID NO 33, LCDR2 of SEQ ID NO 35, LCDR3 of SEQ ID NO 37, HCDR1 of SEQ ID NO 42, HCDR2 of SEQ ID NO 44 and HCDR3 of SEQ ID NO 46.

The inventors of the present disclosure have made intensive research efforts to develop a therapeutic agent capable of effectively killing cancer cells that do not respond to or show resistance to rituximab. As a result, they have found out that an anti-CD20 asymmetric antibody including scFv on one side and Fab on the other side has significantly improved complement-dependent cytotoxicity.

In the present specification, the term "antibody" refers to a protein molecule that acts as a receptor which specifically recognizes an antigen, including an immunoglobulin molecule that has immunological reactivity with a specific antigen, and includes both a polyclonal antibody and a monoclonal antibody.

The antibody includes not only a full-length antibody but also an antigen-binding fragment (antibody fragment) of the antibody molecule. The full-length antibody has a structure having two full-length light chains and two full-length heavy chains, wherein light chain is linked to a heavy chain by a disulfide bond. The heavy chain constant region has gamma (γ), mu (µ), alpha (α), delta (δ) and epsilon (ε) types and has gamma1 (γ1), gamma2 (γ2), gamma3 (γ3), gamma4 (γ4), alpha1 (α1) and alpha2 (α2) subclasses. The light chain constant region has kappa (κ) and lambda (λ) types.

In the present specification, the term "antigen-binding fragment of an antibody" refers to a fragment within the entire antibody molecule, which specifically recognizes an antigen and has the ability of binding to the antigen, and includes a single-domain antibody (sdAb), a single-chain antibody (scFv), Fab, F(ab'), F(ab')₂, Fv, etc. Examples of the fragment include a monovalent fragment consisting of V_{L}, V_{H}, C_{K} (or C_{L}) and C_{H}1 domains (Fab fragment); a bivalent fragment including two Fab fragments linked by a disulfide bridge at the hinge region (F(ab')₂ fragment); a Fd fragment consisting of V_{H} and C_{H}1 domains; a Fv fragment consisting of the V_{L} and V_{H} domains of one arm of an antibody and disulfide-linked Fv (sdFv); a dAb fragment consisting of a V_{H} domain; and an isolated complementarity-determining region (CDR) or a combination of two or more isolated CDRs that may be optionally linked by a linker. In addition, the scFv may be linked by a linker so that the V_{L} and V_{H} regions are paired to form a monovalent molecule constituting a single protein chain. The single-chain antibody is also included in the fragment of an antibody. In addition, the antibody or an fragment of the antibody includes a tetramer antibody including two heavy chain molecules and two light chain molecules; an antibody light chain monomer; an antibody heavy chain monomer; an antibody light chain dimer, an antibody heavy chain dimer; an intrabody; a monovalent antibody; a camel antibody; and a single-domain antibody (sdAb).

Fv is a minimal antibody fragment having only a heavy chain variable region and a light chain variable region, and a recombinant technology for producing an Fv fragment is disclosed in WO 88/10649, WO 88/106630, WO 88/07085, WO 88/07086 and WO 88/09344. A two-chain Fv has a structure in which a heavy chain variable region and a light chain variable region are linked through a noncovalent linkage, and a single-chain Fv includes a heavy chain variable region and a light chain variable region covalently linked to each other via a peptide linker at the C-terminus, thereby forming a dimeric structure as in the two-chain Fv. These antibody fragments may be obtained using proteases (for example, the whole antibody may be restriction-digested with papain to obtain Fab, and may be restriction-digested with pepsin to obtain F(ab')₂ fragments), and may be fabricated using a genetic recombination technique.

In the present specification, the term "heavy chain" refers to both the full-length heavy chain including a variable region domain V_{H} that includes an amino acid sequence sufficient to provide specificity to an antigen and three constant region domains C_{H}1, C_{H}2 and C_{H}3, and fragments thereof. In addition, the term "light chain" in the present specification refers to the full-length light chain including a variable region domain V_{L} that includes an amino acid sequence sufficient to provide specificity to an antigen and a constant region domain C_{K} (or C_{L}), and fragments thereof.

In the present specification, the term "CDR (complementarity-determining region)" refers to an amino acid sequence of a hypervariable region of an immunoglobulin heavy chain or light chain (Kabat et al., Sequences of Proteins of Immunological Interest, 4th Ed., U.S. Department of Health and Human Services, National Institutes of Health (1987)). The heavy chain and light chain include three CDRs (heavy chain CDRH1, CDRH2 and CDRH3 and light chain CDRL1, CDRL2 and CDRL3), respectively. The CDRs provide major contact residues in the binding of an antibody to an antigen or epitope. The CDRs are interspersed with more conserved regions termed framework regions (FRs). Each V_{H} and V_{L} is composed of three CDRs and four FRs, arranged from the amino-terminus to the carboxy-terminus in the order of FR1, CDR1, FR2, CDR2, FR3, CDR3 and FR4.

The antibody or antibody fragment of the present disclosure includes variants having conservative amino acid substitutions in the CDR regions and may include the variants with amino acid sequences described in the attached sequence list as long as CD20 can be recognized specifically. For example, in order to further improve half-life, biocompatibility and other biological properties in addition to the binding affinity of the antibody, additional changes can be made to the amino acid sequence of the antibody. Considering the variations having biologically equivalent activity, the antibody of the present disclosure or a nucleic acid molecule encoding the same is construed to include the sequences having substantial identity to the sequences described in the sequence listings. The substantial identity means that, when the sequence described herein and another sequence are aligned to correspond to each other as much as possible and the aligned sequences are analyzed using an algorithm that is commonly used in the art, the corresponding sequences have at least 61%, more specifically at least 70%, still more specifically at least 80%, and most specifically at least 90%, sequence identity. Methods of alignment for sequence comparison are known in the art. Various methods and algorithms for alignment are disclosed in Smith and Waterman, Adv. Appl. Math. (1981) 2: 482; Needleman and Wunsch, J. Mol. Bio. (1970) 48: 443; Pearson and Lipman, Methods in Mol. Biol. (1988) 24: 307-31; Higgins and Sharp, Gene (1988) 73: 237-44; Higgins and Sharp, CABIOS (1989) 5: 151-3; Corpet et al. Nuc. Acids Res. (1988) 16: 10881-90; Huang et al. Comp. Appl. BioSci. (1992) 8: 155-65; and Pearson et al. Meth. Mol. Biol. (1994) 24: 307-31.

In the present specification, the term "symmetric antibody" refers to an antibody having two light chains and two heavy chains, which has a structure in which one side of the antibody is symmetrical to the other side (e.g., one side and the other side have the same light chain variable region and the same heavy chain variable region).

In the present specification, the term "asymmetric antibody" refers to an antibody with an asymmetric structure unlike the "symmetric antibody". For example, one side may include a fragment including V_{L}-linker-V_{H} (i.e., scFv), and the other side may include a fragment including V_{L}- C_{κ} (or C_{L})-linker-V_{H}- C_{H}1 (i.e., Fab). In addition, one side may include a fragment including Fab, and the other side may include a fragment including Fab and scFv. The asymmetric antibody may additionally include an Fc region. For example, an asymmetric antibody including a fragment including V_{L}-linker-V_{H}-C_{H}2-C_{H}3 on one side and including a fragment including V_{L}- C_{κ} (or C_{L})-linker-V_{H}-C_{H}1-C_{H}2-C_{H}3 another side may be fabricated.

In the present specification, the term "affinity" refers to the binding strength between an antibody or its antigen-binding fragment and an antigen, and may also be expressed as "binding force".

In a specific exemplary embodiment of the present disclosure, the scFv includes a light chain variable region (V_{L}) sequence of SEQ ID NO 51.

In a specific exemplary embodiment of the present disclosure, the scFv includes a heavy chain variable region (V_{H}) sequence of SEQ ID NO 52.

In a specific exemplary embodiment of the present disclosure, the scFv includes a sequence selected from LFR1 of SEQ ID NO 15, LFR2 of SEQ ID NO 17, LFR3 of SEQ ID NO 19, LFR4 of SEQ ID NO 21, HFR1 of SEQ ID NO 23, HFR2 of SEQ ID NO 25, HFR3 of SEQ ID NO 27 and HFR4 of SEQ ID NO 29.

In a specific exemplary embodiment of the present disclosure, the scFv is in the form of V_{L}-linker-V_{H}.

In a specific exemplary embodiment of the present disclosure, the linker may be a combination of Gly and Ser.

In a specific exemplary embodiment of the present disclosure, the Fab includes a light chain variable region (V_{L}) sequence of SEQ ID NO 53.

In a specific exemplary embodiment of the present disclosure, the Fab includes a heavy chain variable region (V_{H}) sequence of SEQ ID NO 54.

In a specific exemplary embodiment of the present disclosure, the Fab includes a sequence selected from LFR1 of SEQ ID NO 32, LFR2 of SEQ ID NO 34, LFR3 of SEQ ID NO 36, LFR4 of SEQ ID NO 38, HFR1 of SEQ ID NO 41, HFR2 of SEQ ID NO 43, HFR3 of SEQ ID NO 45 and HFR4 of SEQ ID NO 47.

In a specific exemplary embodiment of the present disclosure, the Fab includes a C_{κ} (C kappa) sequence of SEQ ID NO 39.

In a specific exemplary embodiment of the present disclosure, the Fab includes a C_{H}1 sequence of SEQ ID NO 48.

In a specific exemplary embodiment of the present disclosure, the Fab is in the form of V_{L}-C_{κ}-linker-V_{H}-C_{H}1.

In a specific exemplary embodiment of the present disclosure, the linker may be a combination of Gly and Ser.

In a specific exemplary embodiment of the present disclosure, the asymmetric antibody of the present disclosure may use a linker consisting of 10 to 100, more specifically 15 to 60, Gly's and Ser's to prevent unwanted pairing of heavy chains.

Specifically, a bispecific antibody of the present disclosure may be in the form wherein CD20-scFv-knob(V_{L}-linker20-V_{H}-C_{H}2-C_{H}3) and CD20-Fab-hole(V_{L}-C_{κ}-linker40-V_{H}-C_{H}1-C_{H}2-C_{H}3) are bound.

The linker20 includes 20 Gly's or Ser's and may be, for example, Gly Gly Gly Gly Ser Gly Gly Gly Gly Ser Gly Gly Gly Gly Ser Gly Ser Gly Gly Ser, although not being limited thereto.

The linker40 includes 40 Gly's or Ser's and may be, for example, Gly Gly Gly Gly Ser Gly Gly Gly Gly Ser Gly Gly Gly Gly Ser Gly Ser Gly Gly Ser Gly Gly Gly Gly Ser Gly Gly Gly Gly Ser Gly Gly Gly Gly Ser Gly Ser Gly Ser Ser, although not being limited thereto.

In another aspect, the present disclosure provides a nucleic acid molecule encoding the antibody or an antigen-binding fragment thereof of the present disclosure described above.

In the present specification, the term "nucleic acid molecule" comprehensively includes DNA (gDNA and cDNA) and RNA molecules, and nucleotides as basic constituent units in the nucleic acid molecule include not only natural occurring nucleotides but also analogues with modified sugars or bases (Scheit, Nucleotide Analogs, John Wiley, New York (1980); Uhlman, and Peyman, Chemical Reviews, 90: 543-584 (1990)). The nucleic acid molecule sequences encoding the heavy chain variable region and the light chain variable region described herein may be modified. Such modification includes addition, deletion, or non-conservative or conservative substitution.

The nucleic acid molecule of the present disclosure is interpreted to include a nucleotide sequence that exhibits substantial identity to the nucleotide sequence described above. The substantial identity means that, when the nucleotide sequence of the present disclosure is aligned with any other sequence to correspond to each other as much as possible and the aligned sequences are analyzed using an algorithm that is ordinarily used in the art, the nucleotide sequences show at least 80% homology, preferably at least 90% homology, and most preferably at least 95% homology.

In another aspect, the present disclosure provides a vector including a nucleic acid molecule encoding the antibody or an antigen-binding fragment thereof of the present disclosure.

In the present specification, the term "vector" refers to a delivery system into which the polynucleotide (nucleic acid) sequence can be inserted for introduction into a cell capable of replicating the polynucleotide sequence. The polynucleotide sequence may be exogenous or heterologous. The vector may be a plasmid, a cosmid vector or a viral vector (retroviral, adenoviral and adeno-associated viral vectors, etc.), although not being limited to. Those skilled in the art can construct the vectors according to standard recombinant techniques (Maniatis, et al., Molecular Cloning, A Laboratory Manual, Cold Spring Harbor Press, Cold Spring Harbor, N.Y., 1988; and Ausubel et al., In: Current Protocols in Molecular Biology, John, Wiley & Sons, Inc., NY, 1994).

In the present specification, the term "expression vector" refers to a vector including a nucleotide sequence encoding at least a portion of a transcribed gene product. In some cases, the RNA molecule is then translated into a protein, a polypeptide or a peptide. The expression vector may include various regulatory sequences. Along with regulatory sequences that regulate transcription and translation, nucleic acid sequences providing other functions may also be included in the vector or the expression vector.

In another aspect, the present disclosure provides a host cell transformed with the vector described above.

In the present specification, the term "cell" includes eukaryotic cells and prokaryotic cells, and refers to any transformable cell that can replicate the vector or can express a gene encoded by the vector. The cell may be transfected, transduced or transformed by the vector, which means a process in which an exogenous polynucleotide (nucleic acid molecule) is delivered or introduced into the host cell. In the present specification, the term "transformation" is used as a meaning that includes transfection and transduction.

Specifically, the (host) cell of the present disclosure includes but is not limited to an insect cell or a mammalian cell. More specifically, the insect cell may be Sf9 cells, and the mammalian cell may be HEK293 cells, HeLa cells, ARPE-19 cells, RPE-1 cells, HepG2 cells, Hep3B cells, Huh-7 cells, C8D1a cells, Neuro2A cells, CHO cells, MES13 cells, BHK-21 cells, COS7 cells, COP5 cells, A549 cells, MCF-7 cells, HC70 cells, HCC1428 cells, BT-549 cells,PC3 cells, LNCaP cells, Capan-1 cells, Panc-1 cells, MIA PaCa-2 cells, SW480 cells, HCT166 cells, LoVo cells, A172 cells, MKN-45 cells, MKN-74 cells, Kato-III cells, NCI-N87 cells, HT-144 cells, SK-MEL-2 cells, SH-SY5Y cells, C6 cells, HT-22 cells, PC-12 cells, NIH3T3 cells, etc.

Specifically, the host cell of the present disclosure is an isolated host cell.

In another aspect, the present disclosure provides a pharmaceutical composition for preventing or treating cancer, which contains the antibody or an antigen-binding fragment thereof of the present disclosure described above, a nucleic acid molecule encoding the antibody or an antigen-binding fragment thereof or a vector including the nucleic acid molecule as an active ingredient.

In another aspect, the present disclosure provides a method for preventing or treating cancer, which includes a step of administering a pharmaceutically effective amount of the antibody or an antigen-binding fragment thereof of the present disclosure described above, a nucleic acid molecule encoding the antibody or an antigen-binding fragment thereof or a vector including the nucleic acid molecule to a subject.

In another aspect, the present disclosure provides a therapeutic use of the antibody or an antigen-binding fragment thereof of the present disclosure described above, a nucleic acid molecule encoding the antibody or an antigen-binding fragment thereof or a vector including the nucleic acid molecule.

In the present specification, the term "prevention" refers to prevention of the occurrence of a disease or a disorder in a subject who has not been diagnosed with the disease or disorder but is likely to suffer from the disease or a disorder.

In the present specification, the term "treatment" means (a) suppression of the development of a disease, a disorder or symptoms; (b) alleviation of a disease, a disorder or symptoms; or (c) elimination of a disease, a disorder or symptoms. When the composition of the present disclosure is administered to a subject, it exhibits a binding force to CD20 equivalent to that of rituximab, and inhibits cancer progression by inhibiting the proliferation of cancer cells through significant complement activity compared to rituximab and/or CDC effect resulting therefrom, or eliminates or alleviates symptoms caused thereby. Accordingly, the composition of the present disclosure may be used as a composition for treating cancer or as a therapeutic adjuvant that enhances therapeutic response together with other pharmacological ingredients. Therefore, in the present specification, the term "treatment" or "therapeutic agent" includes the meaning of "aid of treatment" or "therapeutic adjuvant".

In the present specification, the term "administration" or "administer" refers to direct administration of a therapeutically effective amount of the composition of the present disclosure to a subject so that the same amount is formed in the body of the subject.

In the present disclosure, the term "therapeutically effective amount" means the content of the pharmaceutical composition of the present disclosure, which is sufficient to provide therapeutic or prophylactic effect in a subject, and includes the meaning of "prophylactically effective amount".

In the present specification, the term "subject" includes, without limitation, human, mouse, rat, guinea pig, dog, cat, horse, cow, pig, monkey, chimpanzee, baboon or rhesus monkey. Specifically, the subject of the present disclosure is human.

In a specific exemplary embodiment of the present disclosure, the cancer that can be prevented or treated by the composition of the present disclosure may be CD20-positive cancer.

The CD20-positive cancer includes, for example, blood cancers such as lymphoma, leukemia, etc. and solid cancers such as melanoma, lung cancer, thyroid cancer, breast cancer, colorectal cancer, prostate cancer, head and neck cancer, stomach cancer, liver cancer, bladder cancer, kidney cancer, cervical cancer, pancreatic cancer, ovarian cancer, endometrial cancer, etc. Specifically, it includes non-Hodgkin's lymphoma (NHL), follicular lymphoma, diffuse large B-cell lymphoma, mantle cell lymphoma, chronic lymphocytic leukemia, small lymphocytic lymphoma (SLL), marginal zone lymphoma (MZL), lymphoplasmacytic lymphoma (LDL), Waldenstrom macroglobulinemia (WM), etc., although not being limited thereto.

In another aspect, the present disclosure provides a composition for diagnosing cancer, which contains the antibody or an antigen-binding fragment thereof of the present disclosure described above, a nucleic acid molecule encoding the antibody or an antigen-binding fragment thereof or a vector including the nucleic acid molecule as an active ingredient.

The description of the antibody or an antigen-binding fragment used in the present disclosure and the cancer will be omitted to avoid unnecessary redundancy.

In the present specification, the term "diagnosis" includes determination of a subject's susceptibility to a particular disease, determination of whether a subject currently has a particular disease and determination of the prognosis of a subject suffering from a particular disease.

CD20 is a typical cancer diagnosis marker that shows a higher expression level as compared to a normal level shown in cancer tissues such as B-cell lymphoma and chronic lymphocytic leukemia. The antibody of the present disclosure binding specifically to CD20 can accurately determine the presence of a tumor in a biological sample. In addition, the antibody of the present disclosure can be usefully used to establish a customized treatment strategy according to the nature of cancer since it can predict the treatment sensitivity of a tumor for a CDC drug by accurately measuring the expression level of CD55 in the tumor through various immunoassay methods.

In the present specification, the term "composition for diagnosis" refers to a mixture or a device including the antibody of the present disclosure as a means for measuring the expression level of the CD20 protein to determine whether a subject has cancer or to predict treatment sensitivity to a CDC drug, and may also be expressed as a "diagnostic kit".

In the present specification, the term "biological sample" includes a tissue, a cell, whole blood, serum, plasma, saliva, urine, lymph, spinal fluid, a tissue biopsy sample (brain, skin, lymph node, spinal cord, etc.), a cell culture supernatant, a ruptured eukaryotic cell and a bacterial expression system, although not being limited thereto.

The detection of the CD20 protein in the biological sample may be performed by detecting the formation of an antigen-antibody complex using a colorimetric method, an electrochemical method, a fluorimetric method, a luminometric method, a particle counting method, visual assessment or a scintillation counting method. In the present specification, the term "detection" refers to a series of processes for determining whether a complex has been formed. The detection may be carried out using various labels including enzymes, fluorophores, ligands, luminophores, microparticles or radioisotopes.

The enzymes used as detection labels include, for example, acetylcholinesterase, alkaline phosphatase, β-D-galactosidase, horseradish peroxidase and β-lactamase, the fluorophores include fluorescein, Eu³⁺, Eu³⁺ chelate or cryptate, etc., the ligands include a biotin derivative, etc., the luminophores include acridinium esters, isoluminol derivatives, etc., the microparticles include colloidal gold, colored latex, etc., and the radioisotopes include ⁵⁷Co, ³H, ¹²⁵I, ¹²⁵I-Bonton Hunter reagent, etc.

In a specific exemplary embodiment of the present disclosure, the antigen-antibody complex may be detected by enzyme-linked immunosorbent assay (ELISA). The antibody of the present disclosure may have a detection label and, if it does not have a detection label, the antibody of the present disclosure may be captured and identified by treating with another antibody having a detection label.

In another aspect, the present disclosure provides a method for preparing an anti-CD20 asymmetric antibody with improved complement-dependent cytotoxicity (CDC) or an antigen-binding fragment thereof, which includes:
(a) a step of preparing a single-chain variable fragment (scFv) including LCDR1 of SEQ ID NO 16, LCDR2 of SEQ ID NO 18, LCDR3 of SEQ ID NO 20, HCDR1 of SEQ ID NO 24, HCDR2 of SEQ ID NO 26 and HCDR3 of SEQ ID NO 28; and
(b) a step of preparing an antigen-binding fragment (Fab) including LCDR1 of SEQ ID NO 33, LCDR2 of SEQ ID NO 35, LCDR3 of SEQ ID NO 37, HCDR1 of SEQ ID NO 42, HCDR2 of SEQ ID NO 44 and HCDR3 of SEQ ID NO 46.

In another aspect, the present disclosure provides a method for preparing a nucleic acid molecule encoding an anti-CD20 asymmetric antibody with improved complement-dependent cytotoxicity (CDC) or an antigen-binding fragment thereof, which includes:
(a) a step of preparing a nucleic acid molecule encoding a single-chain variable fragment (scFv) including LCDR1 of SEQ ID NO 16, LCDR2 of SEQ ID NO 18, LCDR3 of SEQ ID NO 20, HCDR1 of SEQ ID NO 24, HCDR2 of SEQ ID NO 26 and HCDR3 of SEQ ID NO 28; and
(b) a step of preparing a nucleic acid molecule encoding an antigen-binding fragment (Fab) including LCDR1 of SEQ ID NO 33, LCDR2 of SEQ ID NO 35, LCDR3 of SEQ ID NO 37, HCDR1 of SEQ ID NO 42, HCDR2 of SEQ ID NO 44 and HCDR3 of SEQ ID NO 46.

The asymmetric antibody of the present disclosure may include an Fc region including first and second subunits that can associate stably. In the present specification, the term "Fc region" refers to the C-terminal region of an antibody heavy chain including at least a portion of the constant region of a full-length antibody, and includes both a wild-type Fc region and a variant Fc region. The Fc region of IgG includes IgG C_{H}2 and IgG C_{H}3 domains. The C_{H}3 domain of the asymmetric antibody of the present disclosure may be a wild-type or variant C_{H}3 domain (e.g., a "protrusion ("knob")" introduced into one chain) or a C_{H}3 domain having a "cavity ("hole")" introduced into the other chain thereof.

In a specific exemplary embodiment of the present disclosure, the asymmetric antibody of the present disclosure may use a knob-into-hole to prevent unwanted pairing of heavy chains. In the "knob-into-hole" method [US 5,731,168; US7,695,936; Ridgway et al., Prot Eng 9, 617-621 (1996); Carter, J Immunol Meth 248, 7-15 (2001)], a protrusion ("knob") is introduced at the interface of a first polypeptide and a corresponding cavity ("hole") is introduced at the interface of a second polypeptide, so that the protrusion can be located in the cavity, in order to promote the formation of a heterodimer and inhibit the formation of a homodimer. The protrusion is introduced at the interface of the first polypeptide by replacing a small amino acid side chain with a larger side chain (e.g., tyrosine or tryptophan). And, the complementary cavity with the same or similar size as the protrusion is formed at the interface of the second polypeptide by replacing a large amino acid side chain with a smaller side chain (e.g., alanine or threonine).

In a specific exemplary embodiment of the present disclosure, the asymmetric antibody of the present disclosure may be in the form wherein the scFv in the form of V_{L}-linker-V_{H}-C_{H}2-C_{H}3 is bound to Fab in the form of V_{L}-C_{κ}-linker-V_{H}-C_{H}1-C_{H}2-C_{H}3.

### [Advantageous Effects]

The features and advantages of the present disclosure may be summarized as follows:
(i) The present disclosure provides an anti-CD20 asymmetric antibody with improved complement-dependent cytotoxicity (CDC), which includes a single-chain variable fragment (scFv) on one side and an antigen-binding fragment (Fab) on the other side, or an antigen-binding fragment thereof.
(ii) Since the asymmetric antibody of the present disclosure exhibits significantly improved complement-dependent cytotoxicity (CDC) compared to an antibody with a symmetric structure, it can be usefully used as an effective therapeutic agent or therapeutic adjuvant which fundamentally eliminates drug resistance in various diseases with induced resistance to rituximab, etc. and significantly improves therapeutic response.

### [Brief Description of Drawings]

FIG. 1 schematically shows IgG and an asymmetric antibody.
FIG. 2 shows an SDS-PAGE analysis result of an asymmetric anti-CD20 antibody.
FIG. 3 shows an ELISA result of analyzing the affinity of an asymmetric anti-CD20 antibody for CD20.
FIG. 4 shows an ELISA result of comparing the complement activity of rituximab and an asymmetric anti-CD20 antibody by measuring the concentration of C4d.
FIG. 5 shows a result of comparing the CDC effect of rituximab and an asymmetric anti-CD20 antibody in Ramos-RR cells showing resistance to rituximab.

### [Best Mode]

Hereinafter, the present disclosure will be described in more detail through examples. These examples are only for describing the present disclosure more specifically and it will be obvious to those having ordinary knowledge in the art that the scope of the present disclosure is not limited by the examples.

### Examples

### Example 1. Cloning of asymmetric anti-CD20 antibody for expression in animal cells

For an asymmetric anti-CD20 antibody, the knob-into-hole technique was used to prevent unwanted pairing of heavy chains and a linker composed of Gly and Ser was used to prevent unwanted binding between light chains. CD20-scFv-knob (V_{L}-linker20-V_{H}-C_{H}2-C_{H}3) and CD20-Fab-hole (V_{L}-C_{κ}-linker40-V_{H}-C_{H}1-C_{H}2-C_{H}3) were cloned, respectively. For the CD20-scFv-knob, PCR was performed using the primer combination shown in Table 1 based on the sequence of rituximab listed in the Drug Data Bank. The amplified genome was treated with *Bss*HII and *Xb*aI (New England Biolabs, UK) enzymes and ligated to a pMAZ vector, a vector for animal cell expression, treated with the same restriction enzymes. The ligated plasmid was transformed into DH5α competent cells (New England Biolabs, UK) by applying heat shock, and plasmids were obtained by culturing the acquired colonies in large scale.

For the CD20-Fab-hole (V_{L}-C_{κ}-linker40-V_{H}-C_{H}1-C_{H}2-C_{H}3), PCR was performed using the primer combination shown in Table 2 based on the sequence of rituximab listed in the Drug Data Bank as for the CD20-scFv-knob. The amplified genome was treated with *Bss*HII and *Xb*aI enzymes and ligated to a pMAZ vector, a vector for animal cell expression, treated with the same restriction enzymes. The ligated plasmid was transformed into DH5α competent cells (New England Biolabs, UK) by applying heat shock, and plasmids obtained by culturing the acquired colonies in large scale were subjected to sequencing analysis (Table 3).

**[Table 1]**

| | Primers | Sequences | |
|---|---|---|---|
| V_{L} | Forward | CGC AGC GAG CGC GCA CTC CCA GAT CGT CCT GAG TCA GAG CC | SEQ ID NO 1 |
| | Reverse | AGA GCC GCC AGA TCC ACT GCC TCC TCC ACC GCT ACC GCC ACC ACC ACT CCC GCC TCC GCC CTT AAT CTC CAA TTT AGT TCC CCC GC | SEQ ID NO 2 |
| V_{H} | Forward | GCA GTG GAT CTG GCG GCT CTC AGG TCC AGC TCC AAC AGC CC | SEQ ID NO 3 |
| | Reverse | GGT GGG CAT GTG TGA GTT TTG TCT GAG CCG CCA GCA CTC ACC GTC ACA GTG GTA C | SEQ ID NO 4 |
| C_{H} | Forward | GAC AAA ACT CAC ACA TGC CCA CC | SEQ ID NO 5 |
| | Reverse | CTC TCC CTG TCC CCG GGT AAA TGA CTA GAA CTA | SEQ ID NO 6 |

| | | | |
|---|---|---|---|
| Primers used for cloning of CD20-scFv-knob | | | |

**[Table 2]**

| | Primers | Sequences | |
|---|---|---|---|
| V_{L}-C_{K} | Forward | CGC AGC GAG CGC GCA CTC CCA GAT TGT CCT GTC TCA GTC TCC TGC | SEQ ID NO 7 |
| | Reverse | CGG CCG CCG TGC GAG ATC TTT TGA TTT CCA GTT TAG TTC CGC CG | SEQ ID NO 8 |
| V_{H} | Forward | CAA GTC CAA CTG CAA CAA CCG GG | SEQ ID NO 9 |
| | Reverse | GGA AGA CCG ATG GGC CCT TGA AGC TAG CGG CGG AAA CCG TTG TGC CAG AG | SEQ ID NO 10 |
| C_{H} | Forward | GCA AGC TTC AAG GGC | SEQ ID NO 11 |
| | Reverse | CTC TCC CTG TCC CCG GGT AAA TGA CTA GAA CTA | SEQ ID NO 12 |

| | | | |
|---|---|---|---|
| Primers used for cloning of CD20-Fab-hole | | | |

**[Table 3]**

| | Amino acids | |
|---|---|---|
| CD20-scFv-knob | QIVLSQSPAILSASPGEKVTMTCRASSSVSYI HWFQQKPGSSPKPWIYATSNLASGVPVRFS GSGSGTSYSLTISRVEAEDAATYYCQQWTSN PPTFGGGTKLEIKGGGGSGGGGSGGGGSG SGGSQVQLQQPGAELVKPGASVKMSCKASG YTFTSYNMHWVKQTPGRGLEWIGAIYPGNG DTSYNQKFKGKATLTADKSSSTAYMQLSSLT SEDSAVYYCARSTYYGGDWYFNVWGAGTTV TVSAGGSDKTHTCPPCPAPELLGGPSVFLFP PKPKDTLMISRTPEVTCVVVDVSHEDPEVKF NWYVDGVEVHNAKTKPREEQYNSTYRVVSV LTVLHQDWLNGKEYKCKVSNKALPAPIEKTIS KAKGQPREPQVYTLPPSRDELTKNQVSLWC LVKGFYPSDIAVEWESNGQPENNYKTTPPVL | SEQ ID NO 13 |
| | DSDGSFFLYSKLTVDKSRWQQGNVFSCSVM HEALHNHYTQKSLSLSPGK | |
| CD20-Fab-hole | QIVLSQSPAILSASPGEKVTMTCRASSSVSYI HWFQQKPGSSPKPWIYATSNLASGVPVRFS GSGSGTSYSLTISRVEAEDAATYYCQQWTSN PPTFGGGTKLEIKRSRTAAAPSVFIFPPSDEQ LKSGTASVVCLLNNFYPREAKVQWKVDNALQ SGNSQESVTEQDSKDSTYSLSSTLTLSKADY EKHKVYACEVTHQGLSSPVTKSFNRGECGG GGSGGGGSGGGGSGSGGSGGGGSGGGGS GGGGSGSGSSQVQLQQPGAELVKPGASVK MSCKASGYTFTSYNMHWVKQTPGRGLEWIG AIYPGNGDTSYNQKFKGKATLTADKSSSTAY MQLSSLTSEDSAVYYCARSTYYGGDWYFNV WGAGTTVTVSAASFKGPSVFPLAPSSKSTSG GTAALGCLVKDYFPEPVTVSWNSGALTSGVH TFPAVLQSSGLYSLSSVVTVPSSSLGTQTYIC NVNHKPSNTKVDKKVEPKSCDKTHTCPPCPA PELLGGPSVFLFPPKPKDTLMISRTPEVTCVV VDVSHEDPEVKFNWYVDGVEVHNAKTKPRE EQYNSTYRVVSVLTVLHQDWLNGKEYKCKV SNKALPAPIEKTISKAKGQPREPQVYTLPPSR DELTKNQVSLSCAVKGFYPSDIAVEWESNGQ PENNYKTTPPVLDSDGSFFLVSKLTVDKSRW | SEQ ID NO 14 |
| | QQGNVFSCSVMHEALHNHYTQKSLSLSPGK | |

| | | |
|---|---|---|
| Amino acid sequences of CD20-scFv-knob and CD20-Fab-hole | | |

### Example 2. Expression in animal cells and purification

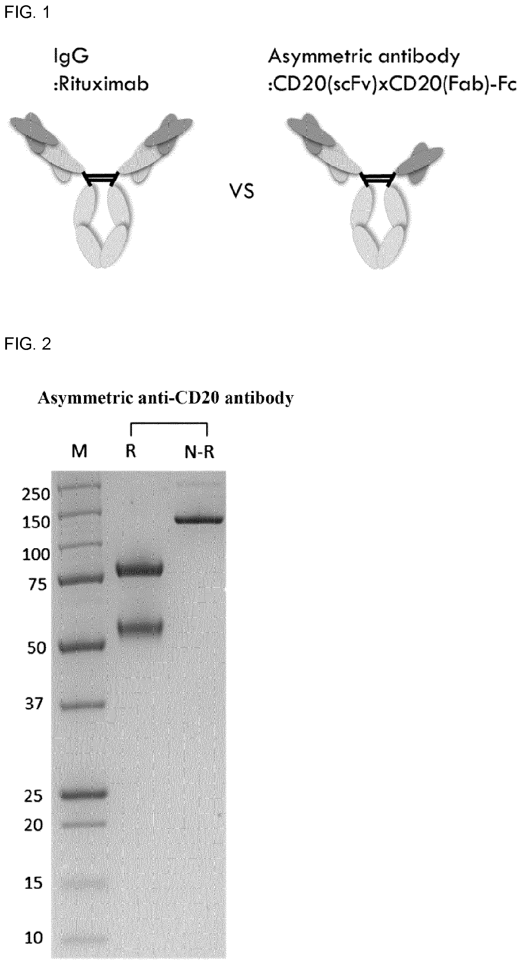

The asymmetric anti-CD20 antibody was transfected into HEK293F cells (Invitrogen, USA) using the plasmids with sequences of SEQ ID NOS 13 and 14 and using polyethylenimine (PEI) (Polysciences, USA) and 150 mM NaCl, and the cells were cultured in a Freestyle 293 expression medium (Invitrogen, USA) at 37 °C for 7 days under the condition of 8% CO₂ and 55% humidity. The cell culture was centrifuged at 4,000 rpm for 10 minutes, and then the supernatant was taken and filtered through a 0.22-µm filter. The filtered supernatant was induced to bind to 1 mL of KappaSelect (GE Healthcare, USA) resin at 4 °C. The bound resin was washed with 10 cv (column volume) of PBS, and the bound antibody was eluted using a 100 mM glycine-HCl (pH 2.7) solution and then neutralized with 1 M Tris-HCl (pH 9.0). After exchanging the buffer with pH 7.2-7.4 PBS, the light chain and heavy chain sizes and purity of the purified antibody were investigated by SDS-PAGE. As a result, the antibody had a molecular weight corresponding to the theoretical value as well as a high purity (FIG. 2).

The asymmetric anti-CD20 antibody was in hetero form with Fab on one side and scFv on the other side. When purified with KappaSelect using the C kappa portion of the Fab, only the desired heterodimer may be obtained while the formation of a knob-knob homodimer or a knob monomer, which are known to be most likely to be produced during the purification of a bispecific antibody (Giese, et al., Bispecific antibody process development: Assembly and purification of knob and hole bispecific antibodies. Biotechnol Prog, 34(2), 397-404).

### Example 3. Confirmation of binding force of asymmetric anti-CD20 antibody to CD20 by ELISA

The affinity of the asymmetric anti-CD20 antibody produced in Example 2 to the antigen CD20 was investigated by indirect ELISA.

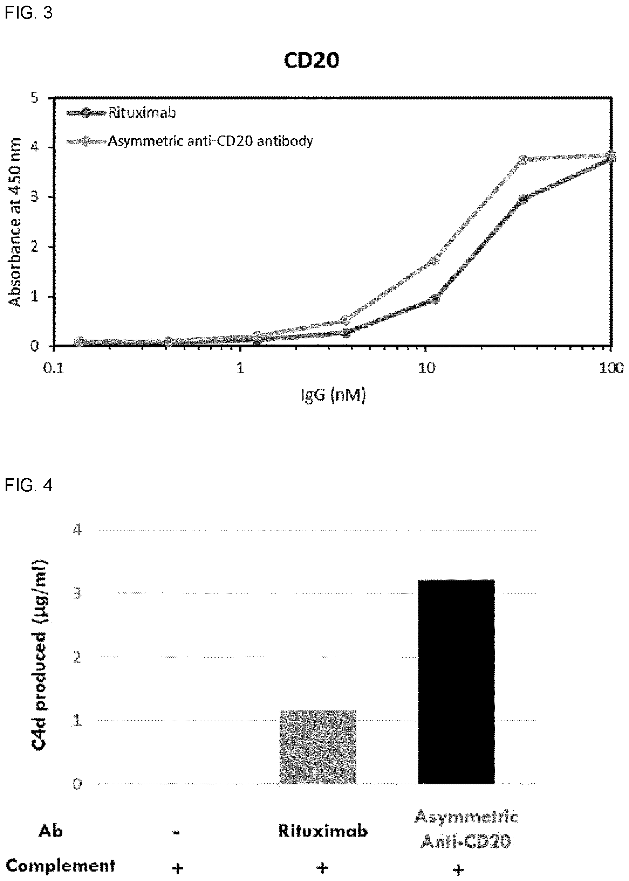

For indirect ELISA, the CD20 ectodomain loop region antigen was diluted in 50 µL of PBS to 1 µg/mL, added to a 96-well immunoplate (Corning, USA) and adsorbed overnight by storing at 4 °C. After reacting with a buffer solution containing 4% skim milk (BD, USA) at room temperature for 1 hour and washing 3 times with a buffer solution containing 0.5% Tween 20 (Amresco, USA), 50 µL of serially diluted antibodies (0.1, 0.3, 1, 3, 11, 33 and 100 nM) were added to each well. After reacting at room temperature for 2 hours so that the antibody can bind to the antigen, it was washed 3 times with a buffer solution containing 0.5% Tween 20 (Amresco, USA). After treating each well with 50 µL of the anti-human immunoglobulin Fc-HRP antibody (Jackson Immunoresearch, USA) diluted to 1:3,000 using a buffer solution containing 2% skim milk (BD, USA), followed by reaction at room temperature for 1 hour, it was washed 3 times with a buffer solution containing 0.5% Tween 20 (Amresco, USA) and reacted with 50 µL of 3,3',5,5'-tetramethylbenzidine (TMB) (ThermoFisher Scientific, USA) for 10 minutes for color development. Absorbance was measured at 450 nm using a spectrophotometer (Biotek, USA) and the result is shown in FIG. 3. The asymmetric anti-CD20 antibody showed a binding affinity to CD20 equivalent to that of rituximab.

### Example 4. Confirmation of complement activity of asymmetric anti-CD20 antibody by C4d ELISA

The complement activity of the asymmetric anti-CD20 antibody produced in Example 2 was investigated by C4d ELISA. C4d is a marker for the activation of the classical complement pathway (Cohen, et al., Pros and cons for C4d as a biomarker. Kidney Int., 81(7), 628-39) and the complement activity can be identified by measuring the concentration of C4d.

First, after reacting 10 µL of 100 µg/mL rituximab or the asymmetric anti-CD20 antibody with 90 µL of 1 mg/mL complement system (Quidel, USA) for 1 hour, followed by dilution to 10-fold, the concentration of C4d was measured using a MicroVue complement C4d fragment EIA kit (Quidel, USA). As shown in FIG. 4, the asymmetric anti-CD20 antibody increased the concentration of C4d as compared to rituximab, whose main mechanism of action is complement-dependent cytotoxicity (CDC), suggesting that the complement activity of the asymmetric anti-CD20 antibody is superior to that of rituximab.

### Example 5. Confirmation of CDC effect of asymmetric anti-CD20 antibody in rituximab-resistant Ramos-RR cells

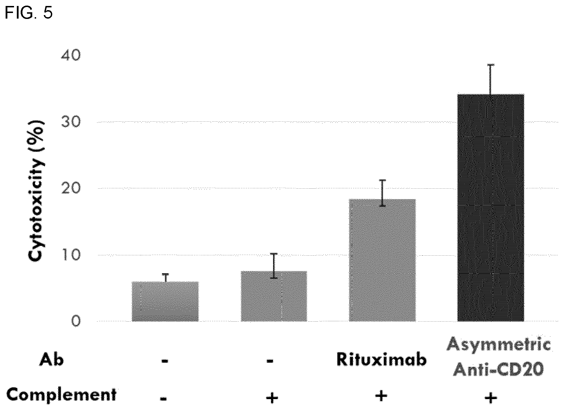

The CDC effect of rituximab and the asymmetric anti-CD20 antibody was investigated in Ramos-RR cells which exhibit resistance to rituximab. 5×10⁵ Ramos-RR cells were treated with 100 µL of rituximab or the asymmetric anti-CD20 antibody, diluted with RPMI supplemented with 20% complement sera human (Sigma, USA), at 20 µg/mL, and then cultured for 2 hours under the condition of 37 °C and 5% CO₂. Subsequently, CDC was investigated by identifying apoptotic cells using an FITC annexin V apoptosis detection kit with 7-AAD (BioLegend, USA) and Attune NxT (ThermoFisher Scientific, USA), a FACS analysis equipment (FIG. 5). The Ramos-RR cells showed low CDC effect for rituximab, but the asymmetric anti-CD20 antibody increased the effect.

This result shows that the asymmetric antibody effectively induces CDC by effectively activating the complement system as compared to the IgG antibody, suggesting that the asymmetric antibody is more advantageous for treatment of cancer the main therapeutic mechanism of which is CDC.

While the specific exemplary embodiments of the present disclosure have been described, those having ordinary knowledge in the art will be able to modify and change the present disclosure variously through addition, change, deletion, etc. It is to be understood that the modifications and changes are included in the scope of the present disclosure.

## Claims

1. An anti-CD20 asymmetric antibody with improved complement-dependent cytotoxicity (CDC) or an antigen-binding fragment thereof, comprising:
(a) a single-chain variable fragment (scFv) comprising LCDR1 of SEQ ID NO 16, LCDR2 of SEQ ID NO 18, LCDR3 of SEQ ID NO 20, HCDR1 of SEQ ID NO 24, HCDR2 of SEQ ID NO 26 and HCDR3 of SEQ ID NO 28; and
(b) an antigen-binding fragment (Fab) comprising LCDR1 of SEQ ID NO 33, LCDR2 of SEQ ID NO 35, LCDR3 of SEQ ID NO 37, HCDR1 of SEQ ID NO 42, HCDR2 of SEQ ID NO 44 and HCDR3 of SEQ ID NO 46.

2. The anti-CD20 asymmetric antibody or an antigen-binding fragment thereof according to claim 1, wherein the scFv comprises a light chain variable region (V_{L}) sequence of SEQ ID NO 51.

3. The anti-CD20 asymmetric antibody or an antigen-binding fragment thereof according to claim 1, wherein the scFv comprises a heavy chain variable region (V_{H}) sequence of SEQ ID NO 52.

4. The anti-CD20 asymmetric antibody or an antigen-binding fragment thereof according to claim 1, wherein the scFv comprises a sequence selected from a group consisting of LFR1 of SEQ ID NO 15, LFR2 of SEQ ID NO 17, LFR3 of SEQ ID NO 19, LFR4 of SEQ ID NO 21, HFR1 of SEQ ID NO 23, HFR2 of SEQ ID NO 25, HFR3 of SEQ ID NO 27 and HFR4 of SEQ ID NO 29.

5. The anti-CD20 asymmetric antibody or an antigen-binding fragment thereof according to claim 1, wherein the scFv is in the form of V_{L}-linker-V_{H}.

6. The anti-CD20 asymmetric antibody or an antigen-binding fragment thereof according to claim 5, wherein the linker is a combination of Gly and Ser.

7. The anti-CD20 asymmetric antibody or an antigen-binding fragment thereof according to claim 1, wherein the Fab comprises a light chain variable region (V_{L}) sequence of SEQ ID NO 53.

8. The anti-CD20 asymmetric antibody or an antigen-binding fragment thereof according to claim 1, wherein the Fab comprises a heavy chain variable region (V_{H}) sequence of SEQ ID NO 54.

9. The anti-CD20 asymmetric antibody or an antigen-binding fragment thereof according to claim 1, wherein the Fab comprises a sequence selected from a group consisting of LFR1 of SEQ ID NO 32, LFR2 of SEQ ID NO 34, LFR3 of SEQ ID NO 36, LFR4 of SEQ ID NO 38, HFR1 of SEQ ID NO 41, HFR2 of SEQ ID NO 43, HFR3 of SEQ ID NO 45 and HFR4 of SEQ ID NO 47.

10. The anti-CD20 asymmetric antibody or an antigen-binding fragment thereof according to claim 1, wherein the Fab comprises a C_{κ} (C kappa) sequence of SEQ ID NO 39.

11. The anti-CD20 asymmetric antibody or an antigen-binding fragment thereof according to claim 1, wherein the Fab comprises a C_{H}1 sequence of SEQ ID NO 48.

12. The anti-CD20 asymmetric antibody or an antigen-binding fragment thereof according to claim 1, wherein the Fab is in the form of V_{L}-C_{κ}-linker-V_{H}-C_{H}1.

13. The anti-CD20 asymmetric antibody or an antigen-binding fragment thereof according to claim 12, wherein the linker is a combination of Gly and Ser.

14. A nucleic acid molecule encoding the anti-CD20 asymmetric antibody or an antigen-binding fragment thereof according to claim 1.

15. A vector comprising the nucleic acid molecule according to claim 14.

16. An isolated host cell transformed with the vector according to claim 15.

17. A pharmaceutical composition for preventing or treating cancer, comprising the anti-CD20 asymmetric antibody or an antigen-binding fragment thereof according to claim 1, a nucleic acid molecule encoding the antibody or an antigen-binding fragment thereof or a vector including the nucleic acid molecule as an active ingredient.

18. A method for preventing or treating cancer, comprising a step of administering a pharmaceutically effective amount of the anti-CD20 asymmetric antibody or an antigen-binding fragment thereof according to claim 1, a nucleic acid molecule encoding the antibody or an antigen-binding fragment thereof or a vector including the nucleic acid molecule to a subject.

19. A therapeutic use of the anti-CD20 asymmetric antibody or an antigen-binding fragment thereof according to claim 1, a nucleic acid molecule encoding the antibody or an antigen-binding fragment thereof or a vector including the nucleic acid molecule.

20. A composition for diagnosing cancer, comprising the anti-CD20 asymmetric antibody or an antigen-binding fragment thereof according to claim 1, a nucleic acid molecule encoding the antibody or an antigen-binding fragment thereof or a vector including the nucleic acid molecule as an active ingredient.

21. A method for preparing an anti-CD20 asymmetric antibody with improved complement-dependent cytotoxicity (CDC) or an antigen-binding fragment thereof, comprising:
(a) a step of preparing a single-chain variable fragment (scFv) comprising LCDR1 of SEQ ID NO 16, LCDR2 of SEQ ID NO 18, LCDR3 of SEQ ID NO 20, HCDR1 of SEQ ID NO 24, HCDR2 of SEQ ID NO 26 and HCDR3 of SEQ ID NO 28; and
(b) a step of preparing an antigen-binding fragment (Fab) comprising LCDR1 of SEQ ID NO 33, LCDR2 of SEQ ID NO 35, LCDR3 of SEQ ID NO 37, HCDR1 of SEQ ID NO 42, HCDR2 of SEQ ID NO 44 and HCDR3 of SEQ ID NO 46.

22. A method for preparing a nucleic acid molecule encoding an anti-CD20 asymmetric antibody with improved complement-dependent cytotoxicity (CDC) or an antigen-binding fragment thereof, comprising:
(a) a step of preparing a nucleic acid molecule encoding a single-chain variable fragment (scFv) comprising LCDR1 of SEQ ID NO 16, LCDR2 of SEQ ID NO 18, LCDR3 of SEQ ID NO 20, HCDR1 of SEQ ID NO 24, HCDR2 of SEQ ID NO 26 and HCDR3 of SEQ ID NO 28; and
(b) a step of preparing a nucleic acid molecule encoding an antigen-binding fragment (Fab) comprising LCDR1 of SEQ ID NO 33, LCDR2 of SEQ ID NO 35, LCDR3 of SEQ ID NO 37, HCDR1 of SEQ ID NO 42, HCDR2 of SEQ ID NO 44 and HCDR3 of SEQ ID NO 46.
